# EUROPEAN PATENT SPECIFICATION

(11) **EP 1 041 990 B1**
(45) Date of publication and mention of the grant of the patent: **28.06.2006**
(21) Application number: 98963828.3
(22) Date of filing: 21.12.1998
(51) Int. Cl.: A61K 31/55, A61K 31/495, A61K 31/47, A61K 31/445, A61P 11/00, A61P 17/00

(54) **COMPOSITION FOR TREATING RESPIRATORY AND SKIN DISEASES, COMPRISING AT LEAST ONE LEUKOTRIENE ANTAGONIST AND AT LEAST ONE ANTIHISTAMINE**
ZUSAMMENSETZUNG ZUR BEHANDLUNG VON ATEMWEGS- UND HAUTERKRANKUNGEN MIT MINDESTENS EINEM LEUKOTRIENANTAGONIST UND MINDESTENS EINEM ANTIHISTAMINIKUM
COMPOSITION POUR LE TRAITEMENT DE MALADIES RESPIRATOIRES ET CUTANEES, COMPRENANT AU MOINS UN ANTAGONISTE DE LEUCOTRIENE ET AU MOINS UN ANTIHISTAMINE

(30) Priority: 23.12.1997 US 68638 P; 19.03.1998 US 78638 P
(43) Date of publication of application: 11.10.2000
(73) Proprietor: SCHERING CORPORATION, Kenilworth, New Jersey 07033-0530 (US)
(72) Inventor: JENSEN, Peder, K., Bedminster, NJ 07921 (US); LORBER, Richard, R., Scotch Plains, NJ 07076 (US); DANZIG, Melvyn, R., Morganville, NJ 07751 (US); MEDEIROS, Paul, T., Easton, PA 18042 (US)
(74) Representative: Adams, Harvey Vaughan John
(86) International application number: PCT/US1998/026223
(87) International publication number: WO 1999/032125

(56) References cited:
- EP-A- 0 565 185
- EP-A- 0 780 127
- WO-A-93/03723
- WO-A-97/28797
- WO-A-97/46243
- WO-A-98/34611
- WO-A-98/48839
- DE-A- 4 203 201

## Description

### BACKGROUND OF THE INVENTION

The present invention relates to compositions for treating allergic rhinitis and other allergic diseases. The products of the 5-lipoxygenase pathway of arachidonic acid metabolism, particularly the leukotrienes, can mediate bronchoconstriction, mucous secretion, airway mucosal edema, chemotaxis and mobilization of cells into the airway in the inflammatory process of asthma. Although useful, leukotriene antagonists, in and of themselves, are not capable of effectively treating the multitude of symptoms that may be associated with disease of the respiratory tract, such as season allergic rhinitis, perennial allergic rhinitis, common colds, sinusitus and concommittant symptoms associated with allergic asthma. The symptoms of such diseases may include sneezing, itching runny nose, nasal congestion, redness of the eye, tearing, itching of the ears or palate, and coughs associated with postnasal drip. It would be highly desirable to enhance the efficacy of such leukotriene antagonists to improve their overall efficacy.

Some specific improved compositions comprising leukotriene antagonists are indicated in the prior art : WO 97/28797 reports combinations of loratadine with various leukotriene antogonists, including montelukast and pranlukast, which combinations are stated to be more efficacious than either agent by itself in treatments of e.g. allergies.

### SUMMARY OF THE INVENTION

The present invention is directed towards a pharmaceutical composition comprising:
i) an effective amount of at least one leukotriene antagonist selected from
   a) montelukast; or
   b) pranlukast;
   or a pharmaceutically acceptable salt thereof; in mixture with
ii) an effective amount of at least one antihistamine which is descarboethoxyloratadine, fexofenadine, ebastine, norastemizole or a pharmaceutically acceptable salt thereof.

Preferably the pharmaceutical composition is designed for oral administration. Preferably the leukotriene antagonist is montelukast and the pharmaceutically acceptable salt of montelukast is montelukast sodium. Also preferred is that the pharmaceutically acceptable salt of monoieukast is 10 milligrams (mg). Most preferably the antihistamine is descarboethoxyloratidine. Preferably, a pharmaceutically acceptable salt of fexofenadine is the hydrochloride salt. Also preferred is that descarboethoxyloratidine is 2.5 to 20 mg, more preferably 5, 7.5 or 10 mg. Preferably, fexofenadine is from 60 to 180 mg. More preferably , the pharmaceutically acceptable salt of montelukast is 10 mg and descarboethoxyloratidine is 5 or 7.5 mg.

Optionally, the pharmaceutical composition can further comprise a third active ingredient which can be:
iii) a decongestant (such as pseudoephedrine), a cough suppressant (such as dextromethorphan), an expectorant/mucolytic (such as guaifenesin), NSAIDs or analgesics (such as aspirin, acetaminophen and phenacetin).

The present invention is useful for treating diseases of the skin, the respiratory tract and/or concomitant symptoms associated therewith, in a mammal, in need of such treatment, comprising administering to said mammal a pharmaceutical composition as described above. Skin diseases include atopic dermatitis, psoriasis and chronic idiopathic urticaria, otherwise known as itchy skin and/or hives. Diseases of the respiratory tract include seasonal allergic rhinitis, perennial allergic rhinitis, common colds, otitis, sinusitus, allergy, asthma, allergic asthma and/or inflammation. Symptoms associated with diseases of the respiratory tract include sneezing, itching and/or runny nose, nasal congestion; redness, tearing or itching of the eye; itching of the ears or palate, shortness of breath, inflammation of the bronchial mucosa, reduced Forced Expiratory Volume In One Second (FEV₁), coughs, rash, hives, itchy skin; headaches, and aches and pains. Descarboethoxyloratidine, fexofenadine, ebastine, norastemizole, or a pharmaceutically acceptable salt thereof and montelukast or pranlukast or pharmaceutically acceptable salt thereof may be administered either either substantially concurrently in separate dosage forms or combined in a unit dosage form as described for the pharmaceutical composition above. Preferably the mammal is a human. Preferably, the separate dosage forms and the unit dosage form of the above pharmaceutical composition are designed for oral administration. Preferably the separate dosage forms and the unit dosage form comprise 5 or 7.5 mg of descarboethoxyloratidine and 10 mg of montelukast sodium.

### DETAILED DESCRIPTION OF THE EMBODIMENTS

### Antihistamines

Descarboethoxyloratidine (DCL) is non-sedating antihistamine, whose technical name is 8-chloro-6,11-dihydro-11-(4-piperidylidene)-5H-benzo[5,6]cyclohepta[1,2]pyridine. This compound is described in Quercia, et al., Hosp. Formul., 28: 137-53 (1993), in U.S. Patent 4,659,716, and in WO 96/20708. DCL is an antagonist of the H-1 histamine receptor protein. The H-1 receptors are those that mediate the response antagonized by conventional antihistamines. H-1 receptors are present, for example, in the ileum, the skin, and the bronchial smooth muscle of man and other mammals. The amount of DCL which can be employed in a unit (i.e. single) dosage form of the present compositions can range from 2.5 to 20 mg, also from 5 to 10 mg, preferably 5 or 7.5 mg.

Fexofenadine (MDL 16,455A) is a non-sedating antihistamine, whose technical name is 4-[1-hydroxy-4-(4-hydroxy-diphenylmethyl)-1-piperidinyl)butyl]-α,α-dimethyl-benzene acetic acid. Preferably the pharmaceutically acceptable salt is the hydrochloride, also known as fexofenadine hydrochloride. The amount of fexofenadine which can be employed in a unit dosage form of the present composition can range from 40 to 200 mg, also from 60 to 180 milligrams, also 120 milligrams.

Ebastine is an antihistamine, whose technical name is 1-[4-(1,1-dimethylethyl)phenyl]-4-[4-(diphenylmethoxy)-1-piperidinyl-1-butanone. CAS90729-43-4. The compound is described in EP134124. The chemical structure for this compound is as follows: 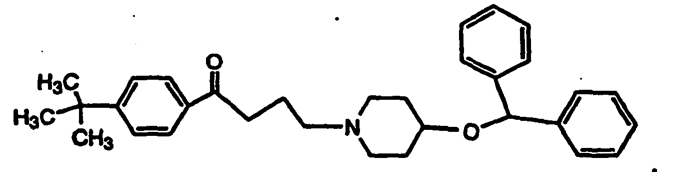 The amount of ebastine which can be employed in a unit dosage form can range from 5 to 20 mg, preferably 10 mg.

Norastemizole is an antihistamine, whose technical name is 1-((4-fluorophenyl)methyl)-N-4-piperidinyl-1H-benzimidazol-2-amine. CAS 75970-99-9. The compound is an active metabolite of astemizole. The chemical structure for this compound is as follows: 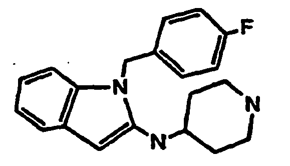 The amount of norastemizole which can be employed in a unit dosage form can range from 5 to 40 mg, also from 10 to 20 mg.

### Leukotriene Antagonists

In addition to and/or in lieu of the amounts cited for any particular compound, the amount of leukotriene antagonist which can be employed in a unit dosage form can range from 5 to 500 milligrams, also from 50 to 300 milligrams, also from 100 to 200 milligrams.

Montelukast is a leukotriene D4 antagonist capable of antagonizing the receptors for the cysteinyl leukotrienes. The technical name of montelukast is [R-(E)]-1-[[[1-[3-[2-(7-chloro-2-quinolinyl)ethenyl]phenyl]-3-[2-(1-hydroxy-1-methylethyl)phenyl]propyl]thio]methyl]cyclopropaneacetic acid. This compound is described in EP 480,717. A preferred pharmaceutically acceptable salt of montelukast is the monosodium salt, also known as montelukast sodium. The amount of montelukast which can be employed in a unit dosage form of the present invention can range from one to 100 milligrams, also from 5 to 20 milligrams, preferably 10 milligrams.

Pranlukast is a leukotriene antagonist described in WO 97/28797 and EP173,516, The technical name for this compound is N-[4-oxo-2-(1H-tetrazol-5-yl)-4H-1-benzopyran-8-yl]-p-(4-phenylbutaoxy)benzamide. The amount of pranlukast which can be employed in a unit dosage from can range from 100 to 700 mg, preferably from 112 to 675 mg; also from 225 mg to 450 mg; also from 225 to 300 mg.

The pharmaceutical compositions of the present invention can be administered depending upon the patient's age, sex, weight and severity of the condition being treated. Generally, the human oral dosage form containing descarboethoxyloratidine, fexofenadine, ebastine, norastemizole, or a pharmaceutically acceptable salt thereof and the leukotriene antagonist can be administered 1 or 2 times per day.

The following table sets forth preferred combinations of a leukotriene antagonist and antihistamine.
Leukotriene Antagonist + Antihistamine
Montelukast + Descarboethoxyloratidine
Pranlukast + Descarboethoxyloratidine
Montelukast + Fexofenadine
Pranlukast + Fexofenadine
Montelukast + Ebastine
Pranlukast + Ebastine
Montelukast + Norastemizole
Pranlukast + Norastemizole

The term "NSAID" as used herein is intended to mean any non-narcotic analgesic non-steroidal anti-inflammatory compound, including the pharmaceutically acceptable salts thereof, falling within one of five structural classes but excluding aspirin, acetaminophen and phenacetin, as follows:
1) The propionic acid derivatives such as ibuprofen, naproxen, flurbiprofen, fenoprofen, ketoprofen, fenbufen and fluprofen;
2) The acetic acid derivatives such as tolmetin sodium sulindac and indomethacin;
3) The fenamic acid derivatives such as mefanamic acid and meclofenamate sodium;
4) The biphenytcarboxylic acid derivatives such as diflunisal and flufenisal; and
5) The oxicams such as piroxicam, sudoxicam and isoxicam.

Analgesics are drugs or compounds that relieve pain, including aspirin, acetaminophen and phenacetin.

In the pharmaceutical compositions and methods of the present invention, the foregoing active ingredients will typically be administered in admixture with suitable pharmaceutical diluents, excipients or carriers (collectively referred to herein as carrier materials) suitably selected with respect to the intended form of administration, i.e. oral tablets, capsules (either solid-filled, semi-solid filled or liquid filled), powders for constitution, oral gels, elixirs, syrups, suspensions, solutions, nasal sprays, opthalmic drops, oral drops, topical creams and the like, and consistent with conventional pharmaceutical practises. For example, for oral adminstration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, mannitol, ethyl alcohol (liquid forms) and the like. Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate.

Additionally, the compositions of the present invention may be formulated in sustained release form to provide the rate controlled release of any one or more of the components or active ingredients to optimize the therapeutic effects, ie. leukotriene antagonism, antihistaminic and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Dosage form - composition descarboethoxyloratidine, fexofenadine, ebastine, norastemizole, or a pharmaceutically acceptable salt thereof and leukotriene antagonist formulated into a delivery system, i.e., tablet, capsule, oral gel, powder for constitution or suspension in association with inactive ingredients.

Capsule - refers to a special container or enclosure made of methyl cellulose, polyvinyl alcohols, or denatured gelatins or starch for holding or containing compositions comprising descarboethoxyloratidine, or fexofenadine and leukotriene antagonist. Hard shell capsules are typically made of blends of relatively high gel strength bone and pork skin gelatins. The capsule itself may contain small amounts of dyes, opaquing agents, plasticizers and preservatives.

Tablet- refers to a compressed or molded solid dosage form containing the active ingredients (descarboethoxyloratidine, or fexofenadine and leukotriene antagonist) with suitable diluents. The tablet can be prepared by compression of mixtures or granulations obtained by wet granulation, dry granulation or by compaction.

Oral gels-refers to descarboethoxyloratidine, or fexofenadine and leukotriene antagonist dispersed or solubilized in a hydrophillic semi-solid matrix.

Powders for constitution refers to powder blends containing descarboethoxyloratidine, or fexofenadine and leukotriene antagonist and suitable diluents which can be suspended in water or juices.

Diluent- refers to to substances that usually make up the major portion of the composition or dosage form. Suitable diluents include sugars such as lactose, sucrose, mannitol and sorbitol; starches derived from wheat, corn rice and potato; and celluloses such as microcrystalline cellulose. The amount of diluent in the composition can range from about 10 to about 90% by weight of the total composition, preferably from about 25 to about 75%, more preferably from about 30 to about 60% by weight, even more preferably from about 12 to about 60%.

Disintegrants - refers to materials added to the composition to help it break apart (disintegrate) and release the medicaments. Suitable disintegrants include starches; "cold water soluble" modified starches such as sodium carboxymethyl starch; natural and synthetic gums such as locust bean, karaya, guar, tragacanth and agar; cellulose derivatives such as methylcellulose and sodium carboxymethylcellulose; microcrystalline celluloses and cross-linked microcrystalline celluloses such as sodium croscarmellose; alginates such as alginic acid and sodium alginate; clays such as bentonites; and effervescent mixtures. The amount of disintegrant in the composition can range from about 2 to about 15% by weight of the composition, more preferably from about 4 to about 10% by weight.

Binders - refers to substances that bind or "glue" powders together and make them cohesive by forming granules, thus serving as the "adhesive" in the formulation. Binders add cohesive strength already available in the diluent or bulking agent. Suitable binders include sugars such as sucrose; starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.

Lubricant - refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.

Glidents - materials that prevent caking and improve the flow characteristics of granulations, so that flow is smooth and unifom. Suitable glidents include silicon dioxide and talc. The amount of glident in the composition can range from about 0.1 % to about 5% by weight of the total composition, preferably from about 0.5 to about 2% by weight.

Coloring agents - excipients that provide coloration to the composition or the dosage form. Such excipients can include food grade dyes and food grade dyes adsorbed onto a suitable adsorbent such as clay or aluminum oxide. The amount of the coloring agent can vary from about 0.1 to about 5% by weight of the composition, preferably from about 0.1 to about 1%.

Bioavailability - refers to the rate and extent to which the active drug ingredient or theraputic moiety is absorbed into the systemic circulation from an administered dosage form as compared to a standard or control.

Conventional methods for preparing tablets are known. Such methods include dry methods such as direct compression and compression of granulation produced by compaction, or wet methods or other special procedures.

## Claims

1. A pharmaceutical composition comprising:
i) an effective amount of at least one leukotriene antagonist selected from
a) montelukast; or
b) pranlukast;
or a pharmaceutically acceptable salt thereof: in mixture with
ii) an effective amount of at least one antihistamine which is descarboethoxytoratadine, fexofenadine, ebastine, norastemizole or a pharmaceutically acceptable salt thereof.

2. The pharmaceutical composition of claim 1 wherein said leukotriene antagonist is montelukast or a phamraceutically acceptable salt thereof.

3. The pharmaceutical composition of claim 2 wherein said antihistamine is descarboethoxylotatadine or a pharmaceutically acceptable salt thereof.

4. The pharmaceutical composition of claim 3 wherein said effective amount of montelukast is 10 milligrams and said effective amount of descarboethoxyloratadine is 5 or 7.5 milligrams.

5. The pharmaceutical composition of claim 1 wherein said leukotrine antagonist is pranlukast or a pharmaceutically acceptable salt thereof.

6. The pharmaceutical composition of claim 5 wherein said antihistamine is desearboethoxyloratadine or a pharmaceutically acceptable salt thereof.

7. The pharmaceutical composition of claim 1 further comprising a third additional active ingredient selected from:
ii))a decongestant, a cough suppressant, an expectorant/mucolytic or an analgesic.

8. The pharmaceutical composition of claim 7 wherein said decongestant is pseudoephedrine.

9. The pharmaceutical composition of claim 7 wherein said cough suppressant is dextromethorphan.

10. The pharmaceutical composition of claim 7 wherein said expectorant/mucolytic is guaifenesin.

11. Use of the composition of any of claims 1-10 for the manufacture of a medicament for treating diseases of the skin, the respiratory tract and/or concomitant symptoms associated therewith in a mammal.

## Patentansprüche

1. pharmazeutische Zusammensetzung, umfassend:
i) eine wirksame Menge von mindestens einem Leukotrien-Antagonisten, der ausgewählt wird aus
a) Montelukast; oder
b) Pranlukast;
oder einem pharmazeutisch wirksamen Salz davon; vermischt mit
ii) einer wirksamen Menge von mindestens einem Antihistamin, das Descarboethoxyloratadin, Fexofenadin, Ebastin, Norastemizol oder ein pharmazeutisch wirksames Salz davon sein kann.

2. Pharmazeutische Zusammensetzung gemäß Anspruch 1, bei der der Leukotrien-Antagonist Montelukast oder ein pharmazeutisch wirksames Salz davon ist.

3. Pharmazeutische Zusammensetzung gemäß Anspruch 2, bei der das Antihistamin Descarboethoxyloratadin oder ein pharmazeutisch wirksames Salz davon ist.

4. Pharmazeutische Zusammensetzung gemäß Anspruch 3, bei der die wirksame Menge an Montelukast 10 mg beträgt und die wirksame Menge an Descarboethoxyloratadin 5 oder 7,5 mg beträgt.

5. Pharmazeutische Zusammensetzung gemäß Anspruch 1, bei der der Leukotrien-Antagonist Pranlukast oder ein pharmazeutisch wirksames Salz davon ist.

6. Pharmazeutische Zusammensetzung gemäß Anspruch 5, bei der das Antihistamin Descarboethoxyloratadin oder ein pharmazeutisch wirksames Salz davon ist.

7. Pharmazeutische Zusammensetzung gemäß Anspruch 1, die ferner einen dritten zusätzlichen Bestandteil enthält, der ausgewählt wird aus:
iii) ein Dekongestionsmittel, ein Hustenmittel, einen schleimlösendes/mucolytieches Mittel oder ein analgetisches Mittel.

8. Pharmazeutische Zusammensetzung gemäß Anspruch 7, bei der das Dekongestionsmittel Pseudoephedrin ist.

9. Pharmazeutische Zusammensetzung gemäß Anspruch 7, bei der das Hustenmittel Dextromethorphan ist.

10. Pharmazeutische Zusammensetzung gemäß Anspruch 7, bei der das schleimlösende/mucolytische Mittel Guaifenesin ist.

11. Verwendung einer Zusammensetzung gemäß irgendeiner der Ansprüche 1-10 für die Herstellung eines Arzneimittels zur Behandlung von Erkrankungen der Haut, der Atemwege und/oder von Begleiterscheinungen, die mit diesen Symptomen bei Säugetieren assoziiert sind.

## Revendications

1. Composition pharmaceutique comprenant ;
i) une quantité efficace d'au moins un antagoniste de leucotriène, choisi parmi
a) le montelukast
b) le pranlukast,
et leurs sels pharmacologiquement admissibles, mélangée avec
ii) une quantité efficace d'au moins un anti-histaminique qui est de la descarboéthoxy-loratadine, de la fexofénadine, de l'ébastine ou du norastémizole, ou l'un de leurs sels pharmacologiquement admissibles.

2. Composition pharmaceutique conforme à la revendication 1, dans laquelle ledit antagoniste de leucotriène est du montelukast ou l'un de ses sels pharmacologiquement admissibles.

3. Composition pharmaceutique conforme à la revendication 2, dans laquelle ledit anti-histaminique est de la descarboéthoxy-loratadine ou l'un de ses sels pharmacologiquement admissibles.

4. Composition pharmaceutique conforme à la revendication 3, dans laquelle ladite quantité efficace de montelukast vaut 10 milligrammes et ladite quantité efficace de descarboéthoxy-loratadine vaut 5 ou 7,5 milligrammes.

5. Composition pharmaceutique conforme à la revendication 1, dans laquelle ledit antagoniste de leucotriène est du pranlukast ou l'un de ses sels pharmacologiquement admissibles.

6. Composition pharmaceutique conforme à la revendication 5, dans laquelle ledit anti-histaminique est de la descarboéthoxy-loratadine ou l'un de ses sels pharmacologiquement admissibles.

7. Composition pharmaceutique conforme à la revendication 1, qui comprend en outre un troisième ingrédient actif supplémentaire (iii) choisi parmi un décongestionnant, un anti-tussif, un expectorant/mucolytique et un analgésique.

8. Composition pharmaceutique conforme à la revendication 7, dans laquelle ledit décongestionnant est de la pseudo-éphédrine,

9. Composition pharmaceutique conforme à la revendication 7, dans laquelle ledit anti-tussif est du dextrométhorphane.

10. Composition pharmaceutique conforme à la revendication 7, dans laquelle ledit expectorant/mucolytique est de la guaïfénésine,

11. Emploi d'une composition conforme à l'une des revendications 1 à 10 en vue de la fabrication d'un médicament destiné au traitement, chez un mammifère, de maladies de la peau ou des voies respiratoires et/ou de symptômes concomitants associés à de telles maladies.
